# EUROPEAN PATENT SPECIFICATION

(11) **EP 3 668 483 B1**
(45) Date of publication and mention of the grant of the patent: **11.02.2026**
(21) Application number: 17921969.6
(22) Date of filing: 18.08.2017
(51) Int. Cl.: A61K 8/891, A61K 8/34, A61K 8/81, A61Q 19/00, A61M 37/00, A61M 35/00, A61N 2/02

(54) **SKIN CARE PRODUCTS**
HAUTPFLEGEPRODUKTE
PRODUITS DE SOINS DE LA PEAU

(43) Date of publication of application: 24.06.2020
(73) Proprietor: The Procter & Gamble Company, Cincinnati, OH 45202 (US)
(72) Inventor: LU, Hao, Singapore 138547 (SG); XU, Zilong, Singapore 138547 (SG)
(74) Representative: P&G Patent Germany
(86) International application number: PCT/CN2017/097959
(87) International publication number: WO 2019/033376

(56) References cited:
- WO-A1-2011/156869
- WO-A1-2011/156869
- WO-A1-2016/044567
- WO-A1-2016/044567
- WO-A1-2019/033160
- WO-A1-2019/033160
- CN-A- 1 988 882
- CN-A- 106 687 102
- W. M. HAYNES, PH.D.: "CRC Handbook of Chemistry and Physics, 97", 2016, CRC PRESS/TAYLOR & FRANCIS, Boca Raton, FL., USA, ISBN: 978-1-4987-5428-6, article DIAMAGNETIC SUSCEPTIBILITY OF SELECTED ORGANIC COMPOUNDS, pages: 3-576 - 3-579, XP002795643

## Description

### FIELD OF THE INVENTION

The present invention relates to skin care products that provide enhanced penetration of a skin care active into skin. Specifically, the present invention relates to the pairing of an applicator and a skin care composition including skin care actives that have particular diamagnetic properties.

### BACKGROUND OF THE INVENTION

Topical skin care compositions containing actives that provide benefits to skin are well known. For example, humectants, particularly glycerin (or glycerol), are known to provide measurable skin regulating benefits. Topical glycerin is known to provide multiple benefits including but not limited to skin moisturization, improving oily skin problems (like pimples, acne etc.), skin smoothening, helping with cell maturation, moisturizing chapped skin, improving mouth ulcers, improving skin tone and reducing wrinkles. Due to its wide range of skin care benefits, glycerin has been extensively used in many topical skin care products and in many forms. For example, glycerin has been used in skin care compositions formulated as creams, lotions, essence formulations, cleansers, toners, substrate masks, and non-substrate mask.

However, effective and optimal delivery of skin care actives, such as glycerin, into skin is an ongoing challenge. Typically, active agents with skin care benefits are introduced to skin via topical application of, for example, creams, lotions, essences, substrate mask and non-substrate mask. However, the actual and perceived benefits of skin care actives such as glycerin are largely dependent on the amount of skin care active that penetrates the top layer of skin and the depth to which it penetrates. There are various factors that limit the amount of active agent that can penetrate skin, and at present, there is little control over the positioning and residency of the active agents following penetration into skin.

The amount of active agent provided in a skin care composition can be increased in various ways, for example, by increasing the amount of active agent in the skin care composition. However, this often leads to compositions that do not have a good sensory feel, increased formulation challenges, stability issues, and increased manufacturing costs.

One approach to improving the efficacy of a skin care active is to use chemical penetration enhancers to facilitate changes in skin permeability, allowing enhanced penetration of the skin care active. However, the use of chemical penetration enhancers can be problematic due to unknown

interaction with the active agent and the potential for adverse side effects such as irritation of skin and mucosal surfaces.

Mechanical approaches to increasing skin penetration of actives have also been explored. For example, one such approach known as iontophoresis utilizes an electrical energy gradient to accelerate a charged active agent(s) across the skin (or other barrier). An example of a device that uses iontophoresis is described in US 7,137,965. However, iontophoresis is only suitable for specific active agents with certain ionic structures and can be injurious to certain dermal barriers due to exchange ion degradation. Additionally, iontophoresis requires the use of intimate electrical contact and adhesive electrodes, which are not suitable for all target surfaces or barriers.

Other techniques for creating mobility and/or direction in the movement of active agent(s) include magneto kinetics and magneto-phoresis. However, these techniques have been difficult to implement due to poor performance, high hardware and energy requirements, and cost. An example of a device that utilizes magnetophoresis is described in US 2009/0093669. While these methods claim to increase the amount of penetration of skin care actives into skin, they still do not provide enhanced penetration in a controlled manner - both in terms of amount of penetration and depth of penetration.

Another example of a device designed to effectively deliver skin care actives is disclosed in WO 2011/156869. The method disclosed includes delivering a skin care agent through a dermal barrier using one or more displaced dipolar magnetic elements. However, this method still does not provide a targeted approach that takes account of the unique properties and targeted benefit areas in skin of different skin care actives.

US2007/053858 A1 discloses a cosmetic skin care product, comprising an applicator comprising a magnetic array, the magnetic array having a first layer of one or more dipolar pairs of alternating magnetic poles with a first layer pitch of between 1mm and 3.5mm, and a first layer magnetic field strength of between 12mT and 30mT; and a skin care composition comprising an effective amount of a vitamin B3 active. In a particular embodiment, the magnetic array further comprises a second layer of at least one dipolar pair of alternating magnetic poles offset from the first layer at an angle of between 1° to 179°, the second layer of magnetic poles having a second layer pitch of between 1mm to 3.5 mm, and a second layer magnetic field strength of between 8 mT and 24 mT, wherein the second layer magnetic field strength is less than or equal to the first layer magnetic field strength.

Accordingly, there is a need to provide a skin care composition, device and/or method that can provide improved penetration of skin care actives into skin in a controlled manner. More specifically there is a need to provide a product and method for enhanced delivery of actives specifically in skin mask compositions that include actives. Further still, there is a need to provide a product and method to enhance the delivery of glycerin in skin mask compositions.

### SUMMARY OF THE INVENTION

According to the invention, there is provided a skin care product, comprising: an applicator including a substrate having a magnetic array embedded therein, the magnetic array including a first layer of at least one dipolar pair of alternating magnetic poles with a pitch of between 1 mm and 3.5 mm and a first layer magnetic field strength of between 12 mT and 30 mT, and a thickness of between 0.2 and 1 mm, wherein the magnetic array further comprises a second layer of at least one dipolar pair of alternating magnetic poles offset from the first layer at an angle of between 1° to 179°, the second layer of magnetic poles having a second layer pitch of between 1mm to 3.5 mm, and a second layer magnetic field strength of between 8 mT and 24 mT, wherein the second layer magnetic field strength is less than or equal to the first layer magnetic field strength ; and a skin mask composition including: glycerin in an amount of between 5% to 15% by weight, at least one silicone oil and/or silicon elastomer in an amount from 0.01% to 10% by weight, fatty alcohol, and at least one thickening agent in an amount from 0.01 to 1% by weight.

### BRIEF DESCRIPTION OF THE DRAWINGS

Embodiments of the invention will hereinafter be described, by way of example, with reference to the accompanying drawings, in which:
FIGS. 1A to 1D are perspective views of applicators of the skin care product described herein;
FIG. 2A shows schematically a conventional bar magnet having a north and a south pole;
FIG. 2B shows schematically a dipolar pair of magnets;
FIGS. 2C and 2D show schematically different arrangements of dipolar pairs in a magnetic array;
FIGS. 3A to 3E illustrate schematically the magnetization and corresponding magnetic field generated in a magnetic array of the skin care product described herein;
FIGS 4A and 4B illustrate schematically different ways of constructing a bi-directional magnetic array of the skin care product described herein; and
FIG. 4C shows schematically a representation of the magnetic field generated by a bi-directional array.

### DETAILED DESCRIPTION OF THE INVENTION

The skin care products disclosed herein exploit the unique diamagnetic property of glycerin to enhance glycerin's penetration into skin. Diamagnetism is the property of an object or material which causes it to create a magnetic field in opposition to an externally applied magnetic field, thus causing a repulsive effect. Surprisingly, it has been discovered that by pairing a specifically tailored magnetic array with glycerin in a skin mask composition containing one or more silicone oils and one or more fatty alcohols, penetration of the active into skin can be enhanced in a controllable way. Utilizing this discovery, it is possible to provide a cosmetic skin care product in which glycerin is delivered into skin to the point where they can provide a better skin care benefit than conventional skin care products.

The skin products disclosed herein provide enhanced penetration of glycerin into skin. Methods of using the present skin products involve the use of a topical skin mask composition in conjunction with an applicator that includes a magnetic array purposefully designed to enhance penetration of glycerin in the composition. The skin mask composition of the present invention may be formulated as a substrate-as well as a non-substrate based mask and preferably formulated in a non-substrate mask form.

### Definitions:

"Apply" or "application", as used in reference to a composition, means to apply or spread the composition onto a surface of keratinous tissue.

"Dermatologically acceptable carrier" means a carrier that may be applied topically to skin or keratinous tissue. The dermatologically acceptable carrier may be in a wide variety of forms such as, for example, simple solutions (water-based or oil-based), solid forms (gels or sticks) and emulsions (water-in-oil or oil-in-water).

"Disposed" refers to an element being located in a particular place or position relative to another element.

"Joined" means configurations whereby an element is directly secured to another element by affixing the element directly to the other element, and configurations whereby an element is indirectly secured to another element by affixing the element to intermediate member(s) that in turn are affixed to the other element.

"Keratinous tissue" refers to keratin-containing layers disposed as the outermost protective covering of mammals which includes, but is not limited to, skin, hair, nails, cuticles, etc.

"Magnetic field" and "magnetic flux density" are used interchangeably herein and refer to the vector field measured in teslas.

"Magnetic material" means a material that can be made into a permanent magnet.

"Non-substrate Mask" means skin care mask formulations without any flexible substrate. These could be in the form of thick paste or cream or essence, typically applied in larger quantities (up to 10 times in comparison to normal skin care products which are non-mask form) on skin. Similar to the substrate mask, the non-substrate mask may also be removed either by peeling and/or washing with water/suitable solvent, or not to be removed from skin after application.

"Pole" refers to the portion of a magnet that exhibits a higher magnetic flux density than the adjacent regions of the magnet. For example, a conventional bar magnet has two poles disposed at opposite ends where the magnetic flux density is highest.

"Regulating skin condition" means improving skin appearance and/or feel, for example, by providing a benefit, such as a smoother appearance and/or feel. Herein, "improving skin condition" means effecting a visually and/or tactilely perceptible positive change in skin appearance and feel. The benefit may be a chronic or acute benefit and may include one or more of the following: reducing the appearance of wrinkles and coarse deep lines, fine lines, crevices, bumps, and large pores; thickening of keratinous tissue (e.g., building the epidermis and/or dermis and/or sub-dermal layers of the skin, and where applicable the keratinous layers of the nail and hair shaft, to reduce skin, hair, or nail atrophy); increasing the convolution of the dermal-epidermal border (also known as the rete ridges), preventing loss of skin or hair elasticity, for example, due to loss, damage and/or inactivation of function skin elastin, resulting in such conditions as elastosis, sagging, loss of skin or hair recoil from deformation; reduction in cellulite; change in coloration to the skin, hair, or nails, for example, under-eye circles, blotchiness (e.g., uneven red coloration due to, for example, rosacea), sallowness, discoloration caused by hyperpigmentation, etc.

"Safe and effective amount" means an amount of a compound or composition sufficient to significantly induce a positive benefit, preferably a positive skin or feel benefit, including independently or in combinations the benefits disclosed herein, but low enough to avoid serious side effects (i.e., to provide a reasonable benefit to risk ratio), within the scope of sound judgment of the skilled artisan).

"Signs of skin aging" include, but are not limited to, all outward visibly and tactilely perceptible manifestations, as well as any macro- or micro-effects, due to keratinous tissue aging. These signs may result from processes which include, but are not limited to, the development of textural discontinuities such as wrinkles and coarse deep wrinkles, fine lines, skin lines, crevices, bumps, large pores, unevenness or roughness; loss of skin elasticity; discoloration (including under-eye circles); blotchiness; sallowness; hyperpigmented skin regions such as age spots and freckles; keratoses; abnormal differentiation; hyperkeratinization; elastosis; collagen breakdown, and other histological changes in the stratum corneum, dermis, epidermis, vascular system (e.g. telangiectasia or spider vessels), and underlying tissues (e.g., fat and/or muscle), especially those proximate to the skin.

"Silicone elastomer" refers to elastomers containing silicone together with carbon, hydrogen and oxygen.

"Silicone oil" as used herein refers to those oils containing at least one silicon atom, and especially containing Si -- O groups.

"Skin" means the outermost protective covering of mammals that is composed of cells such as keratinocytes, fibroblasts and melanocytes. Skin includes an outer epidermal layer and an underlying dermal layer. Skin may also include hair and nails as well as other types of cells commonly associated with skin, such as, for example, myocytes, Merkel cells, Langerhans cells, macrophages, stem cells, sebocytes, nerve cells and adipocytes.

"Skin care" means regulating and/or improving a skin condition. Some nonlimiting examples include improving skin appearance and/or feel by providing a smoother, more even appearance and/or feel; increasing the thickness of one or more layers of the skin; improving the elasticity or resiliency of the skin; improving the firmness of the skin; and reducing the oily, skiny, and/or dull appearance of skin, improving the hydration status or moisturisation of the skin, improving the appearance of fine lines and/or wrinkles, improving skin exfoliation or desquamation, plumping the skin, improving skin barrier properties, improve skin tone, reducing the appearance of redness or skin blotches, and/or improving the brightness, radiancy, or translucency of skin.

"Skin care active" means a compound of combination of compounds that, when applied to skin, provide an acute and/or chronic benefit to skin or a type of cell commonly found therein. Skin care actives may regulate and/or improve skin or its associated cells (e.g., improve skin elasticity; improve skin hydration; improve skin condition; and improve cell metabolism).

"Skin care composition" means a composition that includes a skin care active and regulates and/or improves skin condition.

"Substrate Mask" means skin care mask compositions impregnated on and/or in substrate(s) which are typically flexible. Substrate mask are left on skin for a period of time and are typically removed either by peeling and/or washing with water/suitable solvent.

### Skin care product:

The skin care product described herein includes a skin mask composition containing one or more skin care actives, one of which is glycerin (also known as glycerol), and an applicator that includes a magnetic array tailored to enhance delivery of the glycerin into skin. The skin mask composition and applicator may be packaged and sold together as a single product offering and/or they may be packaged separately to be sold individually. The skin mask composition and the applicator may be packaged in separate packages (e.g., in individual primary packages), which are then joined to one another or placed in a single secondary package. It may be desirable to include indicia on the applicator, the skin care composition and/or their respective package(s), which indicate that the magnetic properties of the array are tailored for use with the skin care composition, for example, to enhance penetration of one or more skin care actives. Indicia suitable for such use are not particularly limited and may include, for example, words, letters, numbers, shapes, colors, pictures and diagrams, which communicate to a consumer that the magnetic array is intended for use with the corresponding cosmetic composition. The indicia may provide a non-verbal communication to a user that the magnetic array enhances penetration of glycerin.

### Applicator:

The cosmetic skin care product described herein includes a suitable applicator for either applying a skin mask composition to a target portion of skin or placing above and/or contacting a target portion of skin to which a skin mask composition has already been applied. The form of the applicator may vary according to the intended target area of application on skin. For example, if the skin mask composition is a whole body mask, then the applicator may be sized and/or shaped to apply the composition to larger surfaces and/or body parts, for example, the legs, arms, abdomen and/or back. The skin mask composition may be intended for use in smaller areas such as the face (e.g., cheeks, forehead, chin, nose, and peri-orbital regions). In such cases, the applicator may be correspondingly shaped and sized to use with smaller surface areas.

The applicator may include a magnetic array as described in more detail below. The magnetic array may be configured to provide a skin contacting surface of the applicator (i.e., the magnetic array is disposed on the applicator such that it is brought into contact with a target skin surface when the applicator is used as intended). Thus, it is important for the magnetic material to be safe for topical use on skin, especially when used with a topical skin mask composition. It may be desirable to select a magnetic material that provides a pleasant feel contacted with skin. For example, the magnetic array may be embedded in the applicator such that the applicator and the magnetic array are a unitary device that provides a smooth, comfortable surface when contacted with skin.

The applicator may include an optional cover placed over at least a portion of the magnetic array and/or skin contacting surface, such that the cover becomes the skin contacting surface of the applicator. The cover may be permanently joined to the applicator, or the cover may be removable, detachable and/or replaceable. It may be desirable for the cover to have a coefficient of friction that is less than that of the magnetic substrate of the magnetic array, which can provide a more desirable user experience when applying a skin care composition with the applicator. The cover may have a dry coefficient of friction (i.e., a coefficient of friction measured without using a composition) that is between 10 and 50% less than the magnetic substrate (e.g., 15%, 20%, 25%, 30%, 35%, 40%, or even 45% less) according to the Friction Test described in the example below. When used to apply a skin care composition, the cover may exhibit a coefficient of friction that is up to 10 times less than the magnetic array (e.g., between 2x and 10x less, 3x and 7x or even between 4x and 6x less).

The optional cover, when included, may be formed from a material that provides a skin contacting surface with better cooling properties than the magnetic substrate. For example, the cover may be formed of a material that has a high thermal conductivity, for example, at least 50 W/mK, 100 W/mK or 200 W/mK. Providing a cover with high thermal conductivity feels cool when contacted with skin. Because the thickness of the cover affects the distance that the magnetic flux density of the magnetic array extends, especially when formed from a non-magnetic material, it is important to ensure that the thickness of the cover does not undesirably inhibit the strength of the applied magnetic field. Suitable cover thicknesses are between 0.1 mm and 5 mm (e.g., between 0.2 and 4 mm, 0.5 and 3 mm, or even between 1 and 2 mm), for non-magnetic materials.

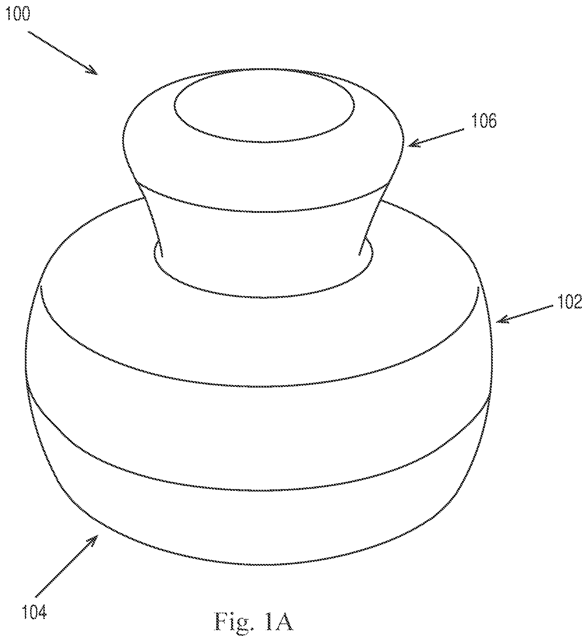

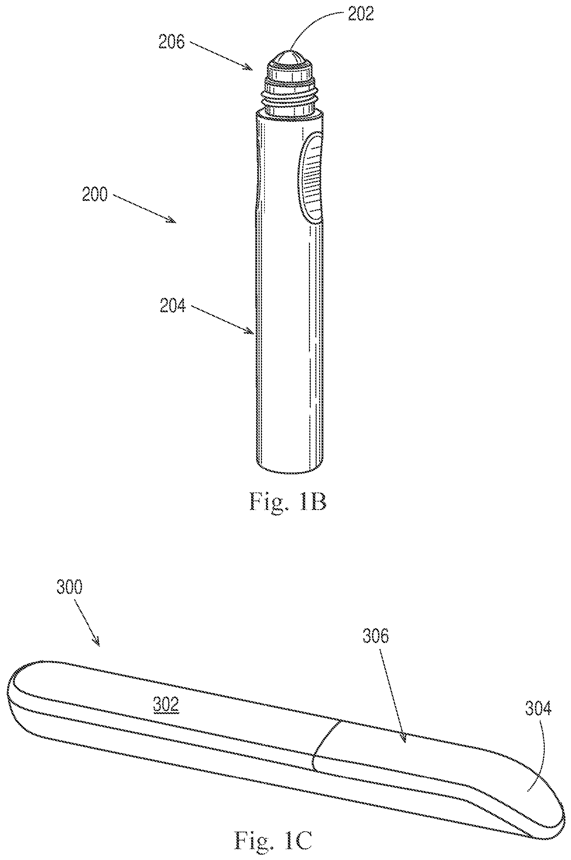

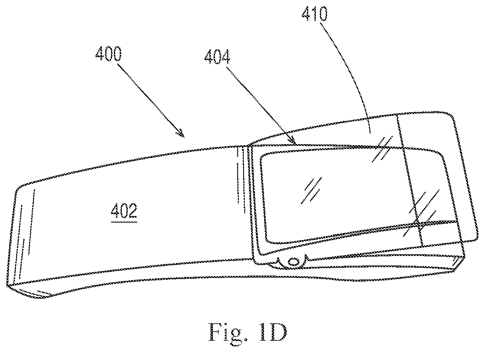

FIGS. 1A, 1B and 1C and 1D show non-limiting examples of applicators 100, 200, 300 and 400, respectively, for use in the present skin care products and methods. The applicator 100 shown in FIG. 1A has a substantially cylindrical base 102 with a skin contact surface 104 extending across the base. A handle 106 extends from the base in a direction substantially perpendicular to the skin contact surface. A magnetic array is disposed inside the base, adjacent to and in parallel with the skin contact surface so that, in use, the magnetic array will be substantially parallel to any surface on which the applicator is used.

The applicator 200 shown in FIG. 1B has a rounded tip 202 that may be suitable for use around the eye. The rounded tip 202 may be integrally formed with a handle 204, or it may be formed as a ball held within a socket 206 at the end of the handle 204. A magnetic array, formed of a flexible substrate is disposed inside the rounded tip 202, such that as the tip 202 is rolled over a surface of skin, the magnetic array will be substantially parallel to the surface of skin. Thus, the tip 202 functions as a cover for magnetic array disposed within the tip 202.

The applicator 300 shown in FIG. 1C has an elongate handle 302 with a skin contacting tip 304 disposed on a skin facing side 306 of the applicator 300. A magnetic array can be disposed inside the applicator 300, adjacent to and in parallel with the skin contacting tip 306, such that the magnetic array will be substantially parallel to any surface on which the applicator 300 is used.

The applicator 400 shown in FIG. 1D includes a removable cover 410. The cover 410 is joined to the skin facing side 404 of the applicator 400 and forms a skin contacting surface of the applicator 400, when used as intended. The cover 410 may be removed and/or replaced, as desired. In some instances, the cover 410 may be removed and reattached, for example, to facilitate cleaning the cover 410 and/or applicator 400. The cover 410 may be disposable. For example, the cover 410 may be removed and discarded after 1 or more uses, but typically less than 10 uses, and replaced with a different cover. The cover 410 may be joined to the applicator 400 by any suitable means known in the art.

The applicator may be used to directly apply a skin mask composition, or used to enhance penetration of skin care actives within a skin mask composition after application of the skin mask composition by some other means, for example, by finger application. For example, the applicator may be designed for movement across the skin's surface - either through manual operation or mechanical means (e.g., a vibrating device) or held in position stationary above a target area of skin to which a skin mask composition has been applied. A vibrating device may include any mechanism, electrical or mechanical, adapted for reciprocal and/or rotational movement of the magnetic material. For example, the magnetic material may be associated with a drive mechanism that is capable of reciprocal movement.

Alternatively, the applicator may be made in the form of, for example, a leave on patch, in which case the applicator may be formed of a woven, flexible fabric. The patch may be formed with an adhesive section such that it can be adhered to a skin's surface following application of the skin mask composition or the skin mask composition may be contained within the patch.

### Magnetic array:

The applicator preferably includes a magnetic array specifically tailored to provide improved penetration of a specific skin care active, such as glycerin. The magnetic array may include selectively magnetized permanent magnets (i.e., materials that create their own persistent magnetic field without an extrinsic power source such as a battery) to generate a magnetic field. The magnets may be formed of any one of numerous known ferromagnetic substrates, including, but not limited to: an iron compound (e.g., a ferrite such as barium ferrite, magnetite, or mild steel), a cobalt material, a strontium material, a barium material, a nickel material, alloys and oxides of these, combinations thereof and the like. The material may have a metalloid component such as boron, carbon, silicon, phosphorous or aluminum. Rare earth material such as neodymium or samarium may also be used.

The magnetic array is preferably designed to work in synergy with the specific diamagnetic properties of glycerin. The overall magnetic field strength of the magnetic array determines the amount of repulsive force induced on glycerin and, as a consequence, the depth within skin to which glycerin is driven, while the pitch of the magnetic poles determines the overall profile of the magnetic field. Use of such a magnetic array together with a composition containing glycerin ensures that the maximum potential amount of glycerin penetrates into a user's skin and is positioned at a layer of skin where it is likely to be most effective.

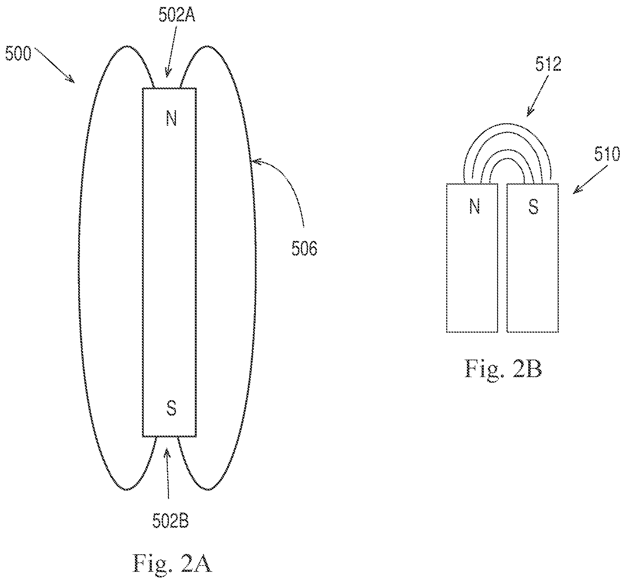

In a conventional bar magnet 500 such as the one illustrated in FIG. 2A, the magnetic field 506 extends between opposite ends 502A and 502B of the magnet 500. In contrast with a conventional bar magnet, the magnetic array(s) described herein are formed of one or more dipole pairs of magnetic elements where magnetic poles of opposite polarity (N and S) are positioned adjacent one another and the magnetic field extends between adjacent opposing poles. For purposes of visualization, a dipole pair 510 may be thought of as a conventional rod magnet that is cleaved at its center and the resulting sections brought together in a north-south (NS), side-by-side configuration.

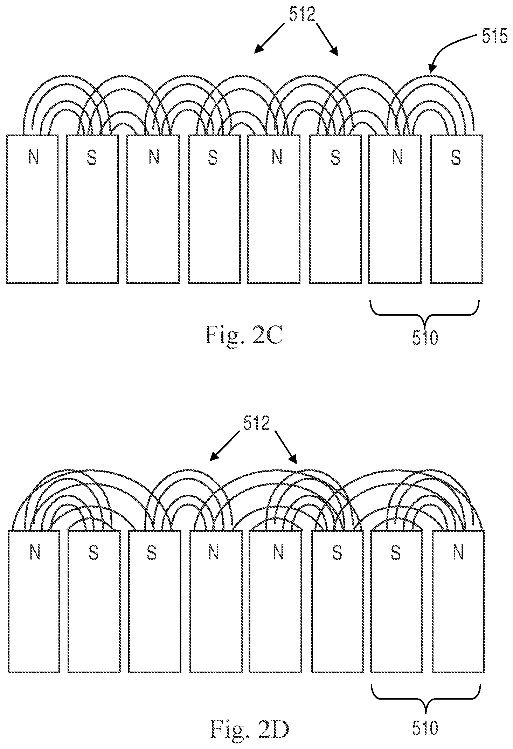

FIGS. 2B, 2C and 2D illustrate examples of magnetic arrays 510. Each of the magnetic arrays in FIGS. 2B, 2C and 2D include one or more dipole pairs 510. Magnetic fields 512 corresponding to the magnetic interaction of the dipole pairs 510 are represented by curved lines. FIG. 2B illustrates a magnetic array with one dipole pair 510 with a single corresponding magnetic field 512, whereas FIGS. 2C and 2D show multiple dipole pairs 510 arranged in series with multiple corresponding magnetic fields 512. When a magnetic array includes multiple dipole pairs 510, such as illustrated in FIGS. 2C and 2D, each dipole pair 510 can be in the same or a different orientation as that of the neighboring pair 510 (e.g., [NS][NS][NS] or [NS][SN][NS] as illustrated schematically in FIGS. 2C and 2D, respectively). In use, the magnetic fields 512 generated by the dipole pairs 510 will induce a magnetic field in a diamagnetic material. The induced magnetic field of the diamagnetic material interacts repulsively with the applied magnetic field 512 of the dipole pairs 510 regardless of the direction of the applied field 512 (i.e., north or south). The magnitude of the repulsive force between magnetic fields 512 of the dipole pairs 510 and the diamagnetic material is determined by the magnetic flux density of the corresponding dipole pair 510 and the diamagnetic susceptibility of the diamagnetic material, in this case the skin care active. Magnetic susceptibility is a dimensionless proportionality constant that indicates the degree of magnetization of a material in response to an applied magnetic field. A negative magnetic susceptibility generally indicates diamagnetism and may be referred to herein as diamagnetic susceptibility. Magnetic flux density is generally greatest at the mid-point 515 between the corresponding poles, and thus the strength of the magnetic field 512 will typically vary across the magnetic array depending on how the array is configured.

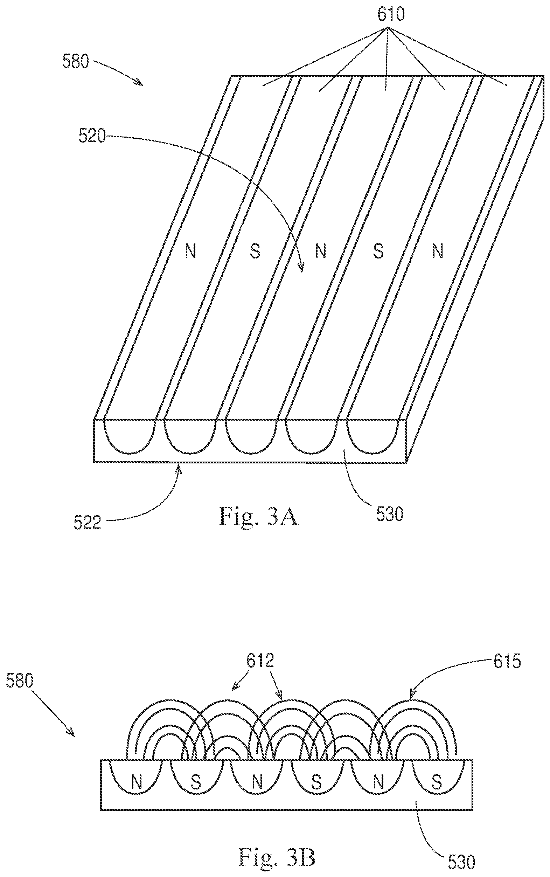

In practice, the substrate 580 used to form a magnetic array for use herein is typically not magnetized evenly throughout. As shown in FIG. 3A, each pole 610 extends from an upper skin facing side 520 of the substrate 580 towards an opposing underside 522 (i.e., through the thickness of the substrate 580). A magnetic return 530 is provided between each adjacent pole 610 and at the second side 522 of the substrate 580. The magnetic return 530 is an unmagnetized area used to integrate the magnetic fields 612 generated by each pole 610 on that side of the substrate 580 and reduce or eliminate the magnetic flux on the second side 522 of the substrate 580, instead diverting it towards the skin facing side 520. The resultant magnetic field 612 extends outward from the first side 520 of the substrate 580, in a direction substantially perpendicular to the surface of the substrate 580, and is strongest at the mid-point 615 between adjacent opposing poles 610.

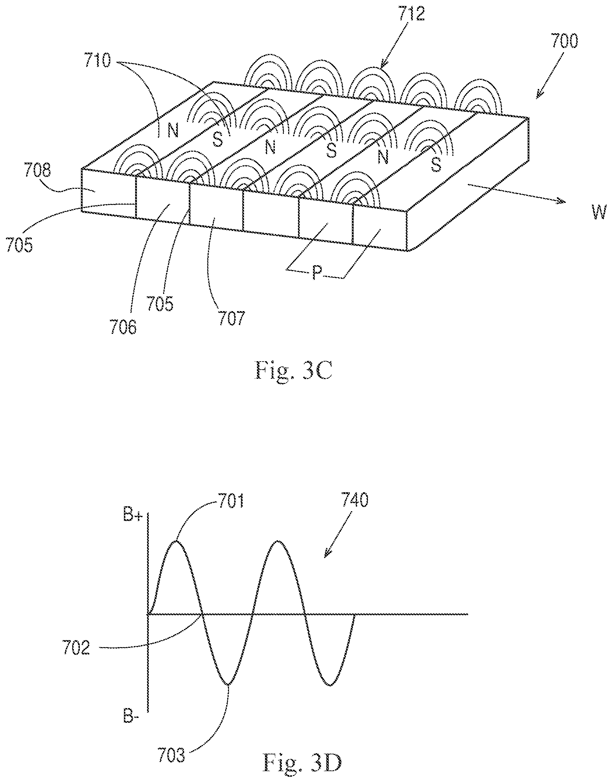

The magnetic array may be formed as a uni-directional array or a multi-directional array. FIG. 3C illustrates an example of a uni-directional array 700. The uni-directional array 700 has north (N) and south (S) poles 710 aligned in parallel to one another in a single layer, as shown in FIG. 3C. Adjacent poles 710 are separated from one another by a pole center-to-center distance P, which defines the pitch of the magnetic array 700.

FIG. 3D illustrates a portion of the magnetic field 712 generated by the magnetic array 700 of FIG. 3C in a direction W that is perpendicular to the alignment of the poles 710. The waveform 740 illustrated in FIG. 3D shows the magnitude of the magnetic field 712 varying regularly between +B and -B in a sinusoidal pattern, which corresponds to the difference in polarity (i.e., direction) of the magnetic field 712. The peaks 701 and troughs 703 of the waveform 740 correspond to the mid-point 705 between adjacent poles 710, and the inflection points 702 of the waveform 740 correspond to the centers of the poles 710. In other words, a first maximum magnetic flux density is represented by peak 701 occurs at a mid-point 705 between a first north pole 708 and an adjacent south pole 706, a minimum magnetic flux density represented by inflection point 702 occurs in the center of the south pole 706, and a second maximum magnetic flux density represented by trough 703 occurs at the mid-point 705 between the south pole 706 and a second north pole 707 adjacent the south pole 706.

The amplitude of the waveform 740 is determined by the choice of magnetic substrate, the thickness or depth of substrate that is magnetized and the distance from the center of a pole 710 to the edge of the pole 710. As the depth of magnetized area of a given substrate material increases, the maximum amplitude of the waveform 740 increases.

The frequency of the waveform 740 is determined by the pitch P of the array. A higher pitch P means that there are fewer magnetic flux density "maximums" per area of substrate, and thus a lower overall magnetic field strength for the array 700. However, a lower pitch P may result in respective poles 710 being packed too closely to one another for any single pole 710 to reach its maximum potential magnetic flux density.

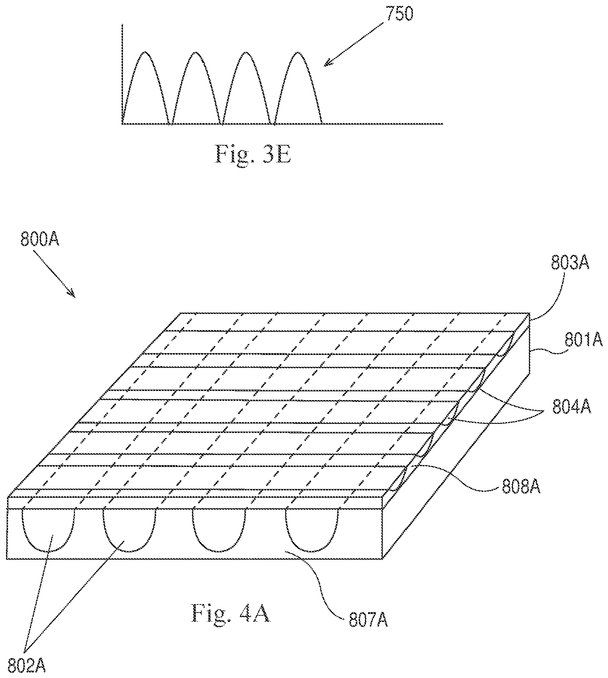

FIG. 3E is an illustration of a waveform 750 representing the repulsive force that would be experienced by a diamagnetic material exposed to the magnetic field 712 in FIG. 3D. As shown by the waveform 750, the induced magnetic field of a diamagnetic material is independent of the direction of the applied magnetic field 712, and thus the change in the magnitude of the repulsive force corresponds to the change in magnitude of the applied magnetic field 712.

The magnetic array may be formed as a multi-directional array, e.g., a bi-directional array, in which multiple layers of parallel poles, which may be configured to play different roles, are juxtaposed at an angle relative to one another to provide multiple magnetic fields that constructively or destructively interfere with one another. For example, a first layer of poles may determine the maximum magnetic field strength, while a second set of poles smooths out the overall profile of the magnetic field, thereby reducing instances of minimum magnetic flux density and ineffectual magnetic field strength. Generally, in a multi-directional array, the magnetic flux density at any one point in the magnetic array will be determined by the combined magnetic flux density of poles of the different layers at that point. This can lead to constructive interference where the resultant magnetic flux density at a point is greater than the magnetic flux density at that point for each individual layer or it may lead to destructive interference where the resultant magnetic flux density at a point is less (sometimes zero) than the magnetic flux density at that point for each individual layer.

FIG. 4A illustrates an example of a bi-directional array 800A, wherein the first and second layers of poles 802A and 804A, respectively, are formed in two separate magnetic substrates 801A and 803A, which are subsequently juxtaposed at an angle offset from one another. The magnetic returns 807A and 808A of both substrates 601 and 603 are positioned to face in the same direction such that the magnetic field generated by both layers of poles 802A and 804A extends away from the magnetic array 800A in the same direction. The layers of poles 802A and 804A may be identical to one another (for example, having the same pitch between adjacent poles and the same maximum field strength), or the two layers 8802A and 8804A may vary in their specific parameters. Where the parameters of the two layers 802A and 804A vary, it is preferable for the layer that is proximal the target diamagnetic material (in FIG. 4A, the second layer 804A) to be formed of a thinner substrate than that of the distal layer (in FIG. 4A, the first layer 802A), otherwise the induced magnetic field of the diamagnetic material will be primarily based on the magnetic field strength of the proximal layer 804A.

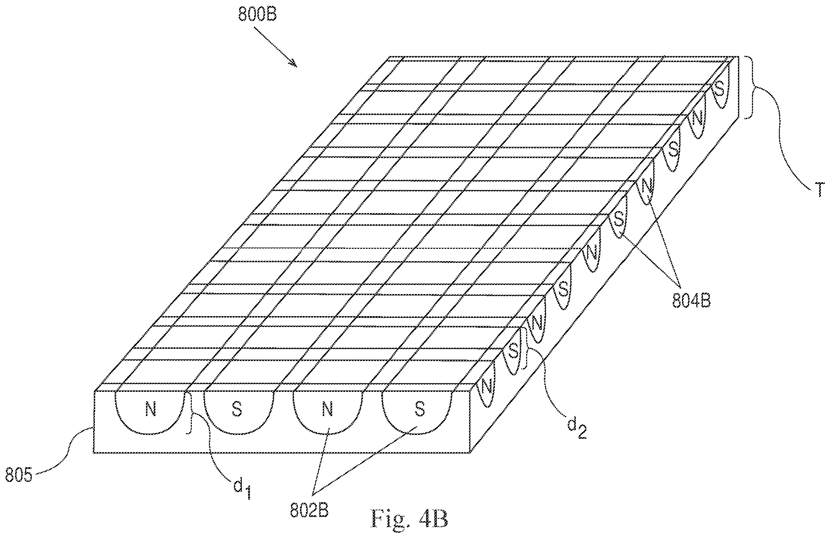

FIG. 4B illustrates an example in which the first layer of poles 802B and the second layer of poles 804B are formed in the same magnetic substrate 805. The configuration shown in FIG. 4B may be provided by first magnetizing the substrate 805 in one direction to form a first layer of parallel aligned north and south poles 802B, and then remagnetizing the substrate 805 in a different direction to form a second layer of parallel aligned north and south poles 804B to effectively form a woven pattern of poles. In FIG. 4B, depth *d2* of poles in the second layer 804B is equal to or less than the depth d1 of poles in the first layer 802B. The depth *d1* of the first layer of poles 802B is typically determined by the thickness T of the magnetic substrate 805.

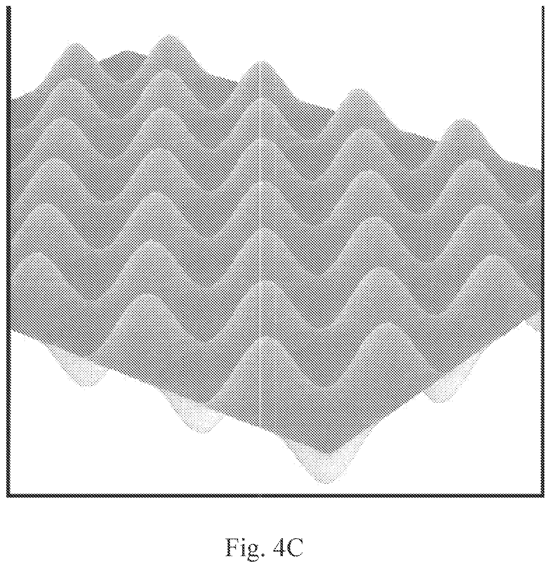

FIG. 4C illustrates a waveform representing the three-dimensional magnetic field of a bidirectional magnetic array. The induced magnetic field of a diamagnetic material is independent of the direction of the magnetic field, and thus all areas of positive and negative magnetic field strength will appear as a repulsive force to a diamagnetic material.

The combined overall magnetic field strength of a magnetic array can be measured after completion of the magnetization process using any known Gaussmeter. For bi-directional magnetic arrays made of two separate substrates, the overall magnetic field strength can be measured first for the respective layers and subsequently for the combined bi-directional magnetic array. In a bi-directional magnetic array, the overall magnetic field strength will approximately equate to the sum of the field strength of the individual layers.

Dipolar pairs of the magnetic substrate may be separated from adjacent dipolar pairs by a magnetically insulating material (i.e., a material with a relatively low magnetic permeability). The magnetic elements may be arranged as individual segments or sections of magnetized ferromagnetic materials. Additionally or alternatively, the magnetic elements may be disposed in or on a solid or semi-solid substrate in which the required magnetic pattern is impressed upon the ferromagnetic particles or elements. The magnetic elements may be rigid elements within the applicator itself or disposed on a suitable substrate and joined to the applicator, for example, with an adhesive. It may be desirable to embed the magnetic elements in a flexible matrix such as rubber or silicone and join the resultant array to a skin facing surface of the applicator.

When pairing a magnetic array with a skin care active such as glycerin, it is important for the magnetic field of the array to be tuned to interact with the diamagnetic susceptibility of the subject skin care active(s). If the magnetic field is improperly configured, for example, if the magnetic flux density is too low or the pitch between adjacent poles too great, there may be little to no magnetic field induced in the diamagnetic materials. Alternatively, if the magnetic flux density is too high, it may induce thermal noise and other forms of molecular entropy or disorder that act against the magnetic enhanced penetration of the skin care active. In some instances, even small departures from the proper configuration of the magnetic array may result in unsatisfactory penetration of the skin care actives.

In one example, a magnetic array is paired with a skin mask composition that includes glycerin in a dermatologically acceptable carrier comprising one or more silicone oils and one or more fatty alcohols. The substrate may be formed of strontium ferrite powder impregnated in a polyvinyl chloride PVC base. A suitable uni-directional array may have a thickness of between 0.2mm to 1mm, 0.3mm to 0.9mm, 0.4mm to 0.8mm, or 0.5mm,to 0.6mm, a pitch (center to center distance between poles) of 1mm to 3.5mm, 1.5mm to 3mm, or 2mm to 2.5mm between adjacent poles, leading to an overall magnetic field strength of between 13mT to 32mT, 14mT to 28mT, 15mT to 25mT, 17.5mT to 22.5mT. In a particularly suitable example of a uni-directional magnetic array, the magnetic array has an overall magnetic field strength of approximately 23mT, a thickness of 0.6mm and a pitch of about 2.1mm (e.g., 12 poles per 25.4mm).

An example of a suitable bi-directional array for enhancing penetration of glycerin into skin may have a first layer thickness of between 0.2mm to 1mm, 0.3mm to 0.9mm, 0.4mm to 0.8mm, or 0.5mm to 0.7mm, a first layer pitch (center to center distance between poles) of 1mm to 3.5mm, 1.5mm to 3mm, or 2mm to 2.5mm between adjacent poles, leading to a first layer magnetic field strength of between 12mT to 30mT, 14mT to 28mT, 15mTto 25mT, or 17.5T to 22.5mT, and a second layer thickness of between 0.05mm to 0.6mm, 0.1mm to 0.4mm, 0.15mm to 0.25, or 0.2mm to 0.25mm, a second layer pitch of 1mm to 3.5mm, 1.25mm to 3mm, or 1.5mm to 2.5mm between adjacent poles, leading to a second layer magnetic field strength of between 8mT to 24mT, 10mT to 22mT, 12mT to 20mT, 14mT to 18mT, or 16mT. The overall magnetic field strength of the bi-directional array may be between 13mT and 32mT. Typically, in a bi-directional array, the second layer magnetic field strength will be less than or equal to the first layer magnetic field strength and/or second layer pitch will be equal to or less than the first layer pitch. The first and second layers of the bi-directional array in this example may be angularly offset by between 1 and 179 degrees, 30 and 160 degrees, 45 and 140 degrees, or 60 to 90 degrees.

In an example of a bi-directional array, the magnetic array has an overall magnetic field strength of 27mT, a first layer thickness of 0.6mm, a first layer pitch of 2.1mm (12 poles per 25.4mm) and a second layer thickness of 0.2mm and second layer pitch of 1.49mm (17 poles per 25.4mm).

The first layer of a bi-directional array may be formed of a uni-directional array.

The method of constructing the magnetic array may involve separately magnetizing two layers of substrate with the two respective layers of poles and arranging the first and second layers of substrate in parallel such that the distal side of the second layer is adjacent the proximal side of the first layer. Alternatively, the method of constructing the magnetic array may involve magnetizing a single ferromagnetic substrate with the poles of the first layer and subsequently magnetizing the same ferromagnetic substrate with poles of the second layer.

### Skin Mask Composition:

A skin mask composition of the present invention may be applied to mammalian keratinous tissue, in particular to human skin. The skin care composition of the present invention maybe formulated as a substrate or a non-substrate mask. The skin mask composition of the present invention may be formulated in a non-substrate mask form. The mask composition may take various forms, including without a substrate. Examples of such masks include sleeping masks, eye masks, body masks, hand masks, foot masks, and the like. The skin mask composition may have a similar appearance to cream or lotion. During mask usage, consumers generally apply 0.5 grams to 5 grams, 1 gram to 2 grams or more of the composition. This is typically more than a normal facial cream and is generally left visible on skin for some time to let the product layer treat the skin. For example, the skin mask may be applied to the skin in an amount such that it is visible on the skin and left on the skin for at least 30 seconds, at least 1 minute, at least 2 minutes, at least 5 minutes, at least 10 minutes, at least 20 minutes or longer. The skin mask may then be removed by the wearer by washing it off, scraping it off, wiping it or any combination of these or other suitable methods.

Skin mask compositions may include glycerin in a dermatological acceptable carrier comprising mixture of one or more silicone oils/elastomers,one or more fatty alcohols and one or more thickener (preferably crosslinked polyacrylate polymer type).

As used herein, "glycerin" means a compound having the formula:

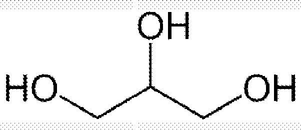

Glycerin also called as 'glycerine' or 'glycerol'. The skin mask composition preferably comprises 0.01 to 20 weight percent of glycerin, and preferably from 5 to 15 weight percent.

The composition may further comprise a dermatologically acceptable carrier. Generally, the amount of the carrier (by weight as a percentage of the composition), is from about 50% to about 99.99%, from about 80% to about 99.9%, from about 90% to about 98% or from about 90% to about 95%.

The carrier may be a variety of forms. For example, the carrier may be in the form of an emulsion, including but not limited to: oil in water, water in oil, and silicone-in-oil-in-water emulsions. Emulsions often have viscosities in the range of about 100 centipoise to about 200,000 centipoise, or in the range from about 20,000 centipoise to about 100,000 centipoise.

Other suitable topical carriers comprise non-aqueous liquid solvents, such as oil, alcohol and silicone (including, but not limited to mineral oil, ethanol, isopropyl alcohol, dimethicone, cyclomethicone and the like); water-based single-phase liquid solvents (e.g. water-alcohol solvent systems); and non-water and water-based single-phase solvent in a thickened form (such as by addition of a suitable glue, resin, wax, polymers, salts and the like, increasing the viscosity of the solvent to form a solid or semi-solid form). Examples of suitable carrier systems are set forth in the following references which are incorporated by reference in their entirety: "Sunscreen Product Formulations Set", "Cosmetics and Toiletries", Vol. 105, 122-139 page (December 1990); "sunscreen product formulations set", "cosmetics and toiletries", Vol. 102, 117-136 page (March 1987); USP4, 960, 764 (Figueroa et al., issued 2 October 1990); with USP4, 254, 105 (Fukuda et al., issued 3 March 1981).

The carrier may comprise water-alcohol system and an oil in water emulsion. When the carrier is an aqueous-alcoholic system, the carrier may contain from about 0% to about 99% of ethanol, isopropanol or mixtures thereof, and about 1% to about 99% of water by weight as a percentage of the composition. The carrier may comprise from about 5% to about 60% of ethanol, isopropanol or mixtures thereof, and about 40% to about 95% of water. A preferred carrier comprises from about 20% to about 50% of ethanol, isopropanol or mixtures thereof, and about 50% to about 80% of water. When the carrier is an oil in water emulsion, the vector may contain any of these emulsions used to prepare the excipient ingredients commonly found. A more detailed discussion of suitable carrier compositions can be found in US 5,605,894 (Blank et al.) and PCT Application WO97/39733 (30 October 1997 Publication, Oblong et al.) which are incorporated by reference herein.

The carrier may comprise one or more silicone oils, one of more of fatty alcohols, and/or one or more thickening agents. The silicone oil may be chosen from non-volatile silicone oils and volatile silicone oils, and mixtures thereof. Suitable silicone oils include polysiloxanes, polyalkyl siloxanes, polyaryl siloxanes, polyalkylaryl siloxanes, polyether siloxane copolymers, and mixtures thereof. The skin mask composition may comprise 0.01 to 20 weight percent of the silicone oils and preferably from 1 to 10 weight percent. Examples of silicone oils are Cyclopentasiloxane, Dimethicone and Dimethiconol blend (one example is a material blend sold by Dow Corning by the name "XIAMETER PMX - 1503 FLUID), Cyclopentasiloxane and Dimethicone Cross (one example is a material blend sold by Dow Corning by the name "Dow Corning 9045 Silicone Elastomer Blend).

"Fatty alcohol" as used herein means a compound having the formula:

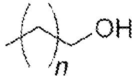

Fatty alcohols are usually high-molecular-weight, straight-chain primary alcohols, derived from natural fats and oils. The fatty alcohols of the present invention have "n" (number of carbons) ranging from 4 to 34. One example of the skin mask composition of the present invention comprises from 0.01% to about 40% weight percent of fatty alcohols. An example of a suitable fatty alcohol is cetyl alcohol. The skin mask composition may include from about 0.1% to about 2% cetyl alcohol.

Thickening agents may be selected from the class of crosslinked polyacrylate polymers. The crosslinked polyacrylate polymer type thickening agents are generally hydrophobically modified. For example, the skin mask composition may include an acrylate such as C10-30 Alkyl Acrylate Crosspolymer type (sold under the trade name Ultrez 21 and Pemulen TR-1). Thickening agents may be included in the composition in an amount of between about 0.01% to about 1%, or 0.2% to 0.5% by weight of the composition. Thickening agents are generally used as rheology modifiers and can provide suspending and stabilizing properties. A skin mask, as described herein, usually has viscosity ranging from 20,000 to 100,000 centipoise as measured with Brookfield RV viscometer T-C Helipath at 25 degree Celsius.

### Methods of Use:

The skin mask product described herein may be used to one or more skin surfaces as part of a user's daily routine. Although not required, the skin mask may be intended primarily for use on facial skin surfaces, including one or more of the cheek, forehead and peri-orbital areas of the face. A consumer may use the skin care product by dispensing a desired amount of the skin mask onto the applicator and then, using the skin contact surface of applicator, applying the mask to a target area of the person's skin. In doing so, the magnetic array located within the applicator can act on the glycerin within the skin mask to increase the volume of glycerin that penetrates into skin. The skin mask may be applied to the applicator manually by the user (for example, by using the applicator to scoop some of the composition out of a tub) and/or the mask may be held in a reservoir provided in the applicator and dispensed automatically onto the skin contact surface of the applicator. Additionally or alternatively, the skin mask composition may be applied directly to a user's skin surface in a normal manner (i.e. by finger application) and the applicator subsequently swept over the target area of skin.

### EXAMPLES

The following examples are given solely for the purpose of illustration and are not to be construed as limiting the invention, as many variations thereof are possible.

### EXAMPLE 1 - mask in vivo skin hydration study #1

An *in vivo* skin hydration study was conducted to establish the hydration effect of using the combination of a skin mask product which contains (i) glycerin 6%, (ii) silicone oils (Cyclopentasiloxane 4%, Dimethicone and Dimethiconol 1.5%, Cyclopentasiloxane and Dimethicone Cross 1%), and (iii) fatty alcohol (Cetyl Alcohol 0.6%) with an applicator comprising a magnetic array which is specified below. The study used both forearms of 20 females as study sites, left and right arms are randomized to be active site (mask applied to target skin surface using an applicator containing a magnetic array of the present invention) and passive study sites (mask applied to target skin surface using traditional finger application). After each application, the study sites were dried by a hairdryer. In this example, the level of skin hydration was measured using a corneometer beforeand after each application and the results were recorded.

### Skin Mask Composition Used:

| Ingredient | | wt % |
|---|---|---|
| Glycerin | Glycerin, USP | 6.00 |
| Fatty alcohol | Cetyl alcohol | 0.60 |
| Silicone oil | Dimethicone and Dimethiconol (Dow Corning-1503) | 1.50 |
| | Cyclopentasiloxane(Xiameter PMX-0245) | 4.00 |
| | Cyclopentasiloxane and Dimethicone Cross polymer (Dow Corning-9045) | 1.00 |
| Thickening Agents | Acrylates/C10-30 Alkyl Acrylate Crosspolymer (Ultrez 21) | 0.36 |
| | Acrylates/C10-30 Alkyl Acrylate Crosspolymer (Pemulen TR-1) | 0.12 |
| Other Ingredients | Purified Water | 86.42 |
| | Butylene Glycol | |
| | Niacinamide,USP | |
| | Isopropyl Isostearate | |
| | Polymethylsilsesquioxane (Tospearl CF600) | |
| | Palmitoyl-Pentapeptide-4 (Promatrixyl) | |
| | Aminomethyl Propanol, 100% | |
| | DMDM Hydantoin and Iodopropynyl Butylcarbamate (Glydant Plus) | |
| | Phenoxyethanol 99 Percent Active | |
| | Benzyl Alcohol, NF | |
| | Dexpanthenol USP | |
| | Ethylhexyl Salicylate (Octisalate), USP | |
| | DL-Alpha Tocopheryl Acetate (Vitamin E Acetate) | |
| | Sodium PEG-7 Olive Oil Carboxylate & Water (Olivem 460) | |
| | Perfume LACEY LIGHT 2012C MOD 1 | |
| | Disodium EDTA (Stay-C 50) | |
| | Sodium Ascorbyl Phosphate | |
| | Mica and Titanium Dioxide - Flamenco Blue | |
| | Red No. 40 | |
| | Yellow 5 | |

### Formula making procedure:

The above materials were split into 7 phases that were pre-mixed and then the phases were mixed together.

| Phase 1 - Main Mix Tank | Procedure |
|---|---|
| Purified Water | Put all material in a stainless steel mixing vessel, heat the contents up to 70-80C, mix with agitator until all materials are mixed evenly. |
| Butylene Glycol | |
| Glycerin, USP | |
| Dimethicone and Dimethiconol (Dow Corning-1503) | |
| Mica and Titanium Dioxide-FLAMENCO BLUE | |

| Phase 2 | Procedure |
|---|---|
| Purified Water | Put all material in a premix stainless steel pot, mix with agitator until all materials are mixed evenly. |
| Disodium EDTA | |
| Acrylates/C10-30 Alkyl Acrylate Crosspolymer (Ultrez 21) | |
| | |

| Phase 3 | Procedure |
|---|---|
| Isopropyl Isostearate | Put all material in a premix stainless steel pot, heat up to 70-80C and mix with agitator until all materials are mixed evenly. |
| Cetyl Alcohol | |
| DL-Alpha Tocopheryl Acetate (Vitamin E Acetate) | |
| Ethylhexyl Salicylate (Octisalate), USP | |
| Acrylates/C10-30 Alkyl Acrylate Crosspolymer (Pemulen TR-1) | |
| | |

| Phase 4 | Procedure |
|---|---|
| Aminomethyl Propanol, 100% | Put all material in a premix stainless steel pot, heat the water to 40-50C and mix manually until all materials are mixed evenly. |
| Purified Water | |

| Phase 5 | Procedure |
|---|---|
| Dexpanthenol, USP | Put all material in a premix stainless steel pot, mix with agitator until all materials are mixed evenly. |
| Purified Water | |
| Niacinamide, USP | |
| Palmitoyl-Pentapeptide-4 (Promatrixyl) | |
| Sodium Ascorbyl Phosphate (Stay-C 50) | |
| Sodium PEG-7 Olive Oil Carboxylate & Water (Olivem 460) | |
| Benzyl Alcohol | |
| Phenoxyethanol | |
| DMDM Hydantoin and Iodopropynyl Butylcarbamate (Glydant Plus) | |

| Phase 6 | Procedure |
|---|---|
| Cyclopentasiloxane and Dimethicone Crosspolymer (Dow Corning-9045) | Put all material in a premix stainless steel pot, mix with agitator until all materials mixed evenly. |
| Cyclopentasiloxane(Xiameter PMX-0245) | |
| Polymethylsilsesquioxane (Tospearl CF600) | |
| Perfume LACEY LIGHT 2012C MOD 1 | |

| Phase 7 | Procedure |
|---|---|
| Purified Water | Put all material in a premix stainless steel pot, mix manually until all materials mixed evenly. |
| Red No. 40 | |
| Yellow 5 | |
| Transfer Phase 3 to Phase 1 Main Mix Tank. Mix with agitator for 5 minutes and mill with high shear milling device for 10 minutes. | |
| Cool Main Mix Tank contents down to 50C while mixing with agitator. Transfer Phase 2, Phase 4, Phase 5, Phase 6 and Phase 7 into Phase 1 Main Mix Tank. Mix for 10 minutes and mill with high shear milling device for 10 minutes. | |
| Continue to cool Main Mix Tank down to 38-40C while mixing with agitator. Mill with high shear milling device for 10 minutes. | |

### Test procedure:

Twenty healthy Chinese subjects, aged between 25 - 55 were recruited.

| | |
|---|---|
| Number of subjects to be recruited at baseline: | 20 |
| Age range of subjects: | 25-55 |
| Sex: | Females |
| Ethnic group: | Chinese |
| Inclusion Criteria: | Overall good health |
| Exclusion Criteria: | Prior skin condition (eczema, psoriasis etc.) |

A 3x4cm area was marked on the left and right forearms of each panelist. The panelists washed their forearms using normal facial cleansers and wiped their arms dry. The panelists then waited atthe test site for 20 minutes until skin normalized prior to the corneometer readings.

The corneometer (Brand:Courage + Khazaka. Mode: MDD4) readings were recorded for both testing sites as initial hydration level. Panelists then dosed 0.05ml mask to both sites, distributed and massage one site with a finger per their normal practice for 20 seconds. The panelists used the applicator of the present invention to distribute and massage the skin mask on their other forearm for 20 seconds. The sites were then dried using a hair drier (Philips ThermoProtect2200 IONIC) to dry at mid warm and mid fan speed mode on both forearms until the skin was completely dry. Corneometer readings from both testing sites were recorded. The delta of corneometer readings before and after were averaged by the 20 data points to evaluate hydration improvement.

The skin hydration measurement for the mask applied by the user's finger was 16.8, while the hydration measurement for the same mask composition applied with the magnet applicator of the present invention was 38.1. The hydration measurement of the skin resulting from use of the mask composition applied with a magnetic wand was surprisingly and significantly higher than the hydration measurement of the skin when applied with the user's finger.

| **Product Name** | **Mask + Wand** | | **Mask + Hand** | |
|---|---|---|---|---|
| **Batch notebook Number** | 20161221 | | 20161221 | |
| **Blinding Code** | A | | B | |
| **Base Size** | 20 | | | |
| | | | | |
| **Delta Immediately After Drying** | 38.10 | **A** | 16.80 | **B** |

The skin hydration was delivered by glycerin as humectant in formula. Surprisingly, as shown in the table, the level of skin hydration attained when using the magnetic applicator of the present invention was more than two times that attained when using a finger alone to apply a skin mask formula.

The applicator used for the above test was configured as follows:
A bi-directional magnetic array (two layers formed on different substrates) was used:
1^{st}-layer:
   a. Substrate: strontium ferrite impregnated in polyvinyl chloride,
   b. Thickness of substrate: 0.6mm, and
   c. Pitch between poles: 2.13mm (12 poles per 25.4mm/0.47 poles per mm) 2^{nd}-layer:
   d. Substrate: strontium ferrite impregnated in polyvinyl chloride,
   e. Thickness of substrate: 0.2mm, and
   f. Pitch between poles: 1.49mm (17 poles per 25.4mm/0.67 poles per mm)
Magnetic array:
   g. Angle between 1^{st} and 2^{nd} layer: 90°, and
   h. Overall magnetic field strength: 24mT

## Claims

1. A skin care product, comprising:
a. an applicator including a substrate having a magnetic array embedded therein, the magnetic array including a first layer, of at least one dipolar pair of alternating magnetic poles with a pitch of between 1 mm and 3.5 mm and a first layer magnetic field strength of between 12 mT and 30 mT, and a thickness of between 0.2 and 1 mm,
wherein the magnetic array further comprises a second layer of at least one dipolar pair of alternating magnetic poles offset from the first layer at an angle of between 1° to 179°, the second layer of magnetic poles having a second layer pitch of between 1mm to 3.5 mm, and a second layer magnetic field strength of between 8 mT and 24 mT, wherein the second layer magnetic field strength is less than or equal to the first layer magnetic field strength; and
b. a skin mask composition including:
i. glycerin in an amount of between 5% to 15% by weight,
ii. at least one silicone oil or silicon elastomer from 0.01% to 20% by weight,
iii. at least one thickening agent from 0.01 to 1% by weight, and
iv. at least one fatty alcohol.

2. The skin care product of claim 1, wherein the first layer has a pitch of 2.1 mm, a thickness of 0.6 mm and a first layer magnetic field strength of 23 mT.

3. The skin care product of claim 1, wherein the substrate comprises a skin facing side and a distal side opposed thereto, and a magnetic return is provided at the distal side.

4. The skin care product of claim 1 having an overall magnetic field strength of the first and second layers between 13 mT and 32 mT, and preferably wherein the second layer pitch is equal to or less than the first layer pitch.

5. The skin care product of claim 1, wherein the first layer and second layer of poles are magnetized on a single substrate having a thickness equivalent to the first layer thickness.

6. The skin care product of claim 1, wherein the first layer and second layer are magnetized on separate substrates, each having a skin facing side and a distal side opposed thereto, and a magnetic return provided at the distal side, wherein the skin facing side of the first layer is arranged adjacent to and parallel with the distal side of the second layer.

7. The skin care product of claim 1, wherein the magnetic array has:
a. a first layer thickness of 0.6 mm, a first layer pitch of 2.1mm;
b. a second layer thickness of 0.2 mm, a second layer pitch of 1.49; and
c. an overall magnetic field strength of the magnetic array of 27 mT.

8. The product of claim 1 wherein the thickening agent is C10-30 Alkyl Acrylate-type crosspolymer and is present in the composition at between 0.01% to 1% by weight of the composition, and preferably wherein silicone oil is present in the composition at between 1% and 10% by weight of the composition, and wherein the silicon oil is preferably selected from the group comprising Cyclopentasiloxane (D5), Dimethicone & Dimethiconol (DC- 1503), Cyclopentasiloxane and Dimethicone Crosspolymer (DC-9045)] and combinations thereof.

9. The skin care product of claim 1 wherein the skin mask is a non-substrate mask, and preferably wherein the skin mask composition has a viscosity in the range from 20,000 to 100,000 centipoise as measured by Brookfield RV viscometer T-C Helipath at 25 degrees Celcius.

## Patentansprüche

1. Hautpflegeprodukt, umfassend:
a. einen Applikator, der ein Substrat, das eine darin eingebettete Magnetbaugruppe aufweist, einschließt, wobei die Magnetbaugruppe eine erste Schicht aus wenigstens einem dipolaren Paar von alternierenden Magnetpolen mit einem Abstand von zwischen 1 mm und 3,5 mm und einer Magnetfeldstärke der ersten Schicht von zwischen 12 mT und 30 mT und einer Dicke von zwischen 0,2 und 1 mm einschließt,
wobei die Magnetbaugruppe ferner eine zweite Schicht aus wenigstens einem dipolaren Paar von alternierenden Magnetpolen umfasst, die von der ersten Schicht in einem Winkel von zwischen 1° bis 179° versetzt sind, wobei die zweite Schicht aus Magnetpolen einen Abstand der zweiten Schicht von zwischen 1 mm bis 3,5 mm und eine Magnetfeldstärke der zweiten Schicht von zwischen 8 mT und 24 mT aufweist,
wobei die Magnetfeldstärke der zweiten Schicht kleiner als oder gleich der Magnetfeldstärke der ersten Schicht ist; und
b. eine Hautmaskenzusammensetzung, die einschließt:
i. Glycerin in einer Menge von zwischen 5 Gew.-% bis 15 Gew.-%,
ii. wenigstens ein Silikonöl oder Siliziumelastomer von 0,01 Gew.-% bis 20 Gew.-%,
iii. wenigstens ein Verdickungsmittel von 0,01 bis 1 Gew.-% und
iv. wenigstens einen Fettalkohol.

2. Hautpflegeprodukt nach Anspruch 1, wobei die erste Schicht einen Abstand von 2,1 mm, eine Dicke von 0,6 mm und eine Magnetfeldstärke der ersten Schicht von 23 mT aufweist.

3. Hautpflegeprodukt nach Anspruch 1, wobei das Substrat eine hautseitige Seite und eine dazu gegenüberliegende distale Seite umfasst und eine Magnetrückführung an der distalen Seite vorgesehen ist.

4. Hautpflegeprodukt nach Anspruch 1, das eine Gesamtmagnetfeldstärke der ersten und der zweiten Schicht zwischen 13 mT und 32 mT aufweist und wobei der Abstand der zweiten Schicht vorzugsweise gleich oder kleiner als der Abstand der ersten Schicht ist.

5. Hautpflegeprodukt nach Anspruch 1, wobei die erste Schicht und die zweite Schicht von Polen auf einem einzigen Substrat magnetisiert sind, das eine Dicke, die der Dicke der ersten Schicht entspricht, aufweist.

6. Hautpflegeprodukt nach Anspruch 1, wobei die erste Schicht und die zweite Schicht auf getrennten Substraten magnetisiert sind, die jeweils eine hautseitige Seite und eine dazu gegenüberliegende distale Seite und eine an der distalen Seite vorgesehene Magnetrückführung aufweisen, wobei die hautseitige Seite der ersten Schicht benachbart zu und parallel zu der distalen Seite der zweiten Schicht angeordnet ist.

7. Hautpflegeprodukt nach Anspruch 1, wobei die Magnetbaugruppe aufweist:
a. eine Dicke der ersten Schicht von 0,6 mm, einen Abstand der ersten Schicht von 2,1 mm;
b. eine Dicke der zweiten Schicht von 0,2 mm, einen Abstand der zweiten Schicht von 1,49; und
c. eine Gesamtmagnetfeldstärke der Magnetbaugruppe von 27 mT.

8. Produkt nach Anspruch 1, wobei das Verdickungsmittel ein Kreuzpolymer einer C10-30-Alkyl-Akrylat-Art ist und in der Zusammensetzung zu zwischen 0,01 Gew.-% bis 1 Gew.-% der Zusammensetzung gegenwärtig ist und wobei Silikonöl in der Zusammensetzung vorzugsweise zu zwischen 1 Gew.-% und 10 Gew.-% der Zusammensetzung gegenwärtig ist und wobei das Silikonöl vorzugsweise ausgewählt ist aus der Gruppe, umfassend Cyclopentasiloxan (D5), Dimethicon & Dimethiconol (DC-1503), Cyclopentasiloxan und Dimethicon-Kreuzpolymer (DC-9045)] und Kombinationen davon.

9. Hautpflegeprodukt nach Anspruch 1, wobei die Hautmaske eine Nichtsubstratmaske ist und wobei die Hautmaskenzusammensetzung vorzugsweise eine Viskosität in dem Bereich von 20.000 bis 100.000 Centipose aufweist, wie durch ein Brookfield RV-Viskosimeter T-C Helipath bei 25 Grad Celsius gemessen.

## Revendications

1. Produit de soin pour la peau, comprenant :
a. un applicateur comportant un substrat ayant un réseau magnétique intégré en lui, le réseau magnétique comportant une première couche d'au moins une paire dipolaire de pôles magnétiques alternés avec un pas compris entre 1 mm et 3,5 mm et une intensité de champ magnétique de première couche comprise entre 12 mT et 30 mT, et une épaisseur comprise entre 0,2 et 1 mm,
dans lequel le réseau magnétique comprend en outre une seconde couche d'au moins une paire dipolaire de pôles magnétiques alternés décalée par rapport à la première couche selon un angle compris entre 1° et 179°, la seconde couche de pôles magnétiques ayant un pas de seconde couche compris entre 1 mm et 3,5 mm, et une intensité de champ magnétique de seconde couche comprise entre 8 mT et 24 mT, dans lequel l'intensité de champ magnétique de seconde couche est inférieure ou égale à l'intensité de champ magnétique de première couche ; et
b. une composition de masque pour la peau comportant :
i. de la glycérine dans une proportion comprise entre 5 % et 15 % en poids,
ii. au moins une huile de silicone ou un élastomère de silicone à 0,01 % à 20 % en poids,
iii. au moins un agent épaississant à 0,01 à 1 % en poids, et
iv. au moins un alcool gras.

2. Produit de soin pour la peau selon la revendication 1, dans lequel la première couche a un pas de 2,1 mm, une épaisseur de 0,6 mm et une intensité de champ magnétique de première couche de 23 mT.

3. Produit de soin pour la peau selon la revendication 1, dans lequel le substrat comprend un côté faisant face à la peau et un côté distal opposé à celui-ci, et un retour magnétique est fourni au niveau du côté distal.

4. Produit de soin pour la peau selon la revendication 1 ayant une intensité de champ magnétique globale de la première et de la seconde couche comprise entre 13 mT et 32 mT, et de préférence dans lequel le pas de seconde couche est égal ou inférieur au pas de première couche.

5. Produit de soin pour la peau selon la revendication 1, dans lequel la première couche et la seconde couche de pôles sont magnétisées sur un substrat unique ayant une épaisseur équivalente à l'épaisseur de première couche.

6. Produit de soin pour la peau selon la revendication 1, dans lequel la première couche et la seconde couche sont magnétisées sur des substrats séparés, chacun ayant un côté faisant face à la peau et un côté distal opposé à celui-ci, et un retour magnétique fourni au niveau du côté distal, dans lequel le côté faisant face à la peau de la première couche est agencé de manière à être adjacent et parallèle au côté distal de la seconde couche.

7. Produit de soin pour la peau selon la revendication 1, dans lequel le réseau magnétique a :
a. une épaisseur de première couche de 0,6 mm, un pas de première couche de 2,1 mm ;
b. une épaisseur de seconde couche de 0,2 mm, un pas de seconde couche de 1,49 ; et
c. une intensité de champ magnétique globale du réseau magnétique de 27 mT.

8. Produit selon la revendication 1, dans lequel l'agent épaississant est un polymère croisé de type C10-30 Alkyl Acrylate et est présent dans la composition à entre 0,01 % et 1 % en poids de la composition, et de préférence dans lequel l'huile de silicone est présente dans la composition à entre 1 % et 10 % en poids de la composition, et dans lequel l'huile de silicone est de préférence choisie dans le groupe comprenant Cyclopentasiloxane (D5), Dimethicone & Dimethiconol (DC-1503), Cyclopentasiloxane et Dimethicone Crosspolymer (DC-9045)] et des combinaisons de ceux-ci.

9. Produit de soin pour la peau selon la revendication 1, dans lequel le masque pour la peau est un masque sans substrat, et de préférence dans lequel la composition de masque pour la peau a une viscosité comprise dans la plage allant de 20 000 à 100 000 centipoises telle que mesurée par un viscosimètre Brookfield RV T-C Helipath à 25 degrés Celsius.
